# EUROPEAN PATENT APPLICATION

(11) **EP 3 705 126 A1**
(43) Date of publication of application: **09.09.2020**
(21) Application number: 19160873.6
(22) Date of filing: 05.03.2019
(51) Int. Cl.: A61K 31/727, A61K 45/06, A61P 43/00

(54) **RONEPARSTAT FOR THE TREATMENT OF AMYLOIDOSIS**

(71) Applicant: Leadiant Biosciences S.p.A., 00144 Roma (IT)
(72) Inventor: NOSEDA, Alessandro, 22012 Cernobbio (CO) (IT)
(74) Representative: Ghirardi, Valeria

(57) **Abstract**

The present invention discloses the heparanase inhibitor Roneparstat or a pharmaceutically acceptable salt, or a hydrate or a solvate thereof for use for the treatment of amyloidosis, in particular primary amyloid light-chain (AL) amyloidosis or secondary amyloid A (AA) amyloidosis.

Also disclosed are pharmaceutical compositions comprising Roneparstat and further active ingredients, selected from cytotoxic agents, proteasome inhibitors, immunomodulatory drugs, topoisomerase inhibitors, kinase inhibitors, steroids and monoclonal antibodies, in particular directed towards highly expressed tumor associated antigens. Roneparstat is also used in a combined therapy with melphalan and/or a proteasome inhibitor or in a proteasome inhibitor and/or melphalan containing regimens.

## Description

### FIELD OF THE INVENTION

The present invention relates to Roneparstat for use for the treatment of amyloidosis.

### BACKGROUND OF THE INVENTION

The term "amyloidosis" encompasses a heterogeneous group of diseases caused by the extracellular deposition of autologous fibrillar proteins, which aggregate into a three-dimensional ß-lamina disposition that impairs normal organ function.

The main subtypes of systemic amyloidosis are primary AL amyloidosis, secondary amyloid A (AA) amyloidosis, familial amyloidosis, and ß2-microglobulin-related amyloidosis. AA amyloidosis is probably the most common type of amyloidosis worldwide, given that most reported cases from developing countries are associated with underlying infections. Systemic AL amyloidosis, on the other hand, which was previously known as primary amyloidosis, is the most prevalent type in developed countries.

Amyloid deposits in AA amyloidosis are composed mainly of the serum amyloid A (SAA) protein, an apolipoprotein of high-density lipoproteins that serves as a dynamic acute phase reactant. Treatment strategies target formation of amyloid proteins (Real De Asua et al, 2014).

AL amyloidosis results from extra-cellular deposition of fibril-forming monoclonal immunoglobulin (Ig) light chains (LC) (most commonly of lambda isotype) usually secreted by a small plasma cell clone. The key event in the development of AL amyloidosis is the change in the secondary or tertiary structure of an abnormal monoclonal LC, which results in instable conformation. This conformational change is responsible for abnormal folding of the LC, rich in β leaves, which assemble into monomers that stack together to form amyloid fibrils.

Treatment of AL amyloidosis is based on chemotherapy, aimed at controlling the underlying plasma clone that produces amyloidogenic LC.

There is currently no treatment able to accelerate the catabolism of AL amyloid deposits. All available therapeutic strategies work by reducing the production of the amyloidogenic precursor, thus enabling the body to slowly eliminate existing deposits (Desport et al., 2012; Mahmood et al., 2014).

Novel strategies are under search to target amyloid deposits (Merlini, 2013).

Besides amyloid, heparan sulfate proteoglycan (HSPG), is commonly found in most amyloid deposits, suggesting that HS/HSPG may be functionally involved in the pathogenesis of amyloidosis. HS or HSPG is found to interact with a number of amyloid proteins, displaying a promoting effect on amyloid fibrilization in vitro. Co-deposits of HS/HSPG with amyloid have been detected in most of the systemic and localized amyloidosis diseases. It has been proposed that HS may facilitate formation of the nidus and/or protofilament around which amyloid fibrillogenesis takes place and impart stability to the amyloid fibril in vivo. This notion was supported by the marked increase in fibrillogenesis in vitro exerted by HS or its analogue heparin (a mammalian polysaccharide sharing similar structure with HS) on various amyloidogenic polypeptides. HS has also been found to affect processing of amyloid precursor proteins (APPs) by acting on proteolytic enzymes and to alter cytotoxicity of amyloid proteins. So, HS and HSPG may be functionally engaged in amyloid deposition through common mechanisms (Zhang, 2010).

Eprodisate is a low molecular-weight molecule that is similar to heparan sulfate. By binding competitively to GAG union sites, it inhibits polymerization of amyloid fibrils and prevents the stabilization of amyloid deposits. This further supports that heparan sulfate and glycosaminoglycan (GAG) chains from the extracellular matrix are crucial for amyloid fibrillogenesis. (Real De Asua et al, 2014).

HS is evidenced to be involved in a number of steps/phases of amyloidogenesis. *In vivo* evidence for functional roles of HS in amyloidogenesis has been obtained from experiments performed with a transgenic mouse model overexpressing human heparanase, an endo-b-d-glucuronidase that catalyzes the hydrolytic cleavage of HS. The liver, expressing high level of heparanase, of the transgenic mice became resistant to the systemic AA amyloidosis. Therefore, it was proposed that HS may function as a scaffold to facilitate amyloid deposition (Zhang, 2010). It was also shown in mice that heparanase-induced fragmentation of HS led to a reduced amyloid burden (Jendresen, 2014). Therefore, heparanase was supposed to play a role in the pathological process of AA amyloidosis, even if its role is not yet clearly defined (Zhang, 2010; Jendresen, 2014; Wang, 2012).

Inhibition of interactions within GAGs and amyloid fibrils is proposed as a promising therapeutic approach in AL amyloidosis (Mahmood et al., 2014).

In view of the above, a treatment for amyloidosis, in particular for AA amyloidosis and AL amyloidosis is still strongly needed.

In particular, a treatment able to target and reduce the amyloid deposits would be highly desirable.

### DESCRIPTION OF THE INVENTION

It has now been surprisingly found that Roneparstat, a known inhibitor of heparanase, has a beneficial effect on the regression of amyloid deposits. Indeed, a regression of the amyloid score was observed in mouse models of amyloidosis treated with Roneparstat, with respect to controls.

It is therefore one object of the present invention Roneparstat for use for the treatment of amyloidosis.

Amyloidosis is preferably primary AL amyloidosis or secondary amyloid A (AA) amyloidosis.

In a preferred embodiment, Roneparstat is used for the treatment of amyloidosis in a combined therapy with a chemotherapeutic agent, in particular melphalan or a proteasome inhibitor, or in a proteasome inhibitor and/or melphalan containing regimens. In the combined therapy, Ronepartstat may be administered in any way of carrying out the regimen. For example, Roneparstat can be administered before, concomitantly or after the administration of the other chemotherapeutic agent, in particular a proteasome inhibitor and/or melphalan. The way and timing of the administration, either concomitant or sequential, will be determined by the skilled person, according to the therapeutic need of the patient.

Another object of the present invention is a pharmaceutical composition comprising Roneparstat and at least one pharmaceutically acceptable vehicle and/or excipient for use for the treatment of amyloidosis.

In a preferred embodiment, said pharmaceutical composition can also contain one or more further active ingredients, for example chemotherapeutic agents, such as melphalan or a proteasome inhibitor, preferably selected from the group consisting of bortezomib, carfilzomib and ixazomib.

### Detailed description of the invention

The present invention will be now disclosed in detail also by means of examples and figures.

### Figures

Figure 1 shows fall of the circulating SAA (Serum AA) concentration in mice actively depositing amyloid (after amyloid enhancing factor - AEF - injection), because of the high rate at which the protein is sequestered in amyloid.
Figure 2 shows the experimental protocol to demonstrate the effect of the present invention in amyloid deposition in mouse transgenic model.
Figures 3-4 show the effect of the present invention in amyloid load in spleen of transgenic mouse model.
Figures 5-6 show the effect of the present invention in amyloid load in liver of transgenic mouse model.
Figure 7 shows the experimental protocol to demonstrate the effect of the present invention in amyloid regression in mouse transgenic model.
Figure 8 shows the effect of the present invention in amyloid regression in spleen of transgenic mouse model.
Figure 9 shows the effect of the present invention in amyloid regression in liver of transgenic mouse model.

Roneparstat (INN) (previously also designated as ¹⁰⁰NA,ROH or SST0001 or G4000) is a modified heparin derivative that is 100% N-desulphated, N-reacetylated and glycol split. These modifications abolish any clinically relevant anticoagulant activity, but enhance the inhibition of heparanase.

Roneparstat has the following formula (I): 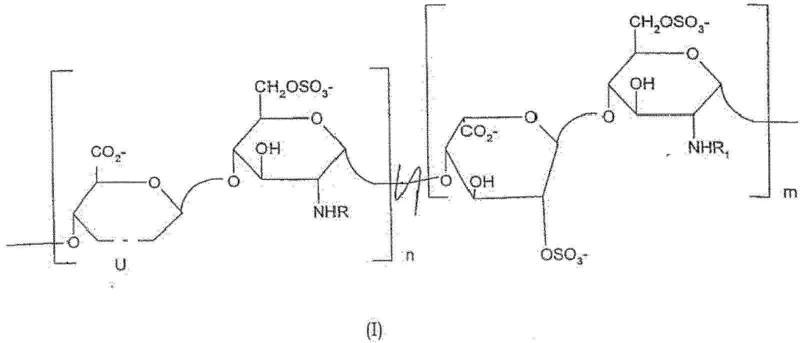 where the U ring is: 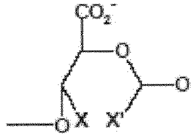 X and X' are the -CH₂-D group, where D is hydroxy;
R and R₁ are an acetyl residue;
n and m, which can be the same or different, may vary from 1 to 40; the sum of m+n ranges from 6 to 40; the m:n ratio ranges from 10:2 to 1:1,
the symbol 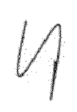 indicates that units marked m and n are statistically distributed along the polysaccharide chain and are not necessarily in sequence.

For a complete description of this compound and its manufacturing process, reference can be made to the published patents EP2343077, US7781416 (see ¹⁰⁰NA,RO-H, paragraph bridging columns 24 and 25) and US8067555. Reference can also be made to US8222231 and to Ritchie JP, Ramani VC, Ren Y, Naggi A, Torri G, Casu B, et al. Roneparstat, also referred to as SST0001, inhibits myeloma growth and angiogenesis via disruption of the heparanase/syndecan-1 axis (Clin Cancer Res 2011;17:1382-93).

Roneparstat can also be used in the form of a pharmaceutically acceptable salt, or hydrate or include other solvents used for crystallization, also known as solvates.

What is meant by pharmaceutically acceptable salt of compound is any salt of the latter with an acid that does not give rise to toxic or side effects.

Non-limiting examples of such salts are: chloride, bromide, orotate, aspartate, acid aspartate, acid citrate, magnesium citrate, phosphate, acid phosphate, fumarate and acid fumarate, magnesium fumarate, lactate, maleate and acid maleate, oxalate, acid oxalate, pamoate, acid pamoate, sulphate, acid sulphate, glucose phosphate, tartrate and acid tartrate, glycerophosphate, mucate, magnesium tartrate, 2-amino-ethanesulphonate, magnesium 2-amino-ethanesulphonate, methanesulphonate, choline tartrate, trichloroacetate, and trifluoroacetate.

A list of FDA-approved pharmaceutically acceptable salts is given in the publication Int. J. of Pharm. 33 (1986), 201-217.

According to the present invention, Roneparstat is for use in the treatment of amyloidosis. For amyloidosis it is intended a disease caused by the extracellular deposition of autologous fibrillar proteins.

Amyloidosis is preferably selected from the group consisting of: primary amyloid light-chain (AL) amyloidosis, secondary amyloid A (AA) amyloidosis, familial amyloidosis, and ß2-microglobulin-related amyloidosis.

Preferably, Roneparstat is for use in the treatment of primary amyloid light-chain (AL) amyloidosis or of secondary amyloid A (AA) amyloidosis.

A pharmaceutical composition comprising Roneparstat for use in the treatment of amyloidosis is also within the scope of the present invention.

The pharmaceutical composition for the use according to the invention comprising Roneparstat can also contain one or more further active ingredients, for example chemotherapeutic agents. For example, said chemotherapeutic agent can be selected from the group consisting of cytotoxic agents, proteasome inhibitors, immunomodulatory drugs, topoisomerase inhibitors, kinase inhibitors and monoclonal antibodies, in particular directed towards highly expressed tumor associated antigens. When said chemotherapeutic agent is a cytotoxic agent, it is preferably selected from the group consisting of melphalan, irinotecan, rituximab and lapatinib; it can also be an alkylating agent, such as cyclophosphamide. When said chemotherapeutic agent is a proteasome inhibitor, it is preferably selected from the group consisting of bortezomib, carfilzomib and ixazomib. When said chemotherapeutic agent is an immunomodulatory agent, it is preferably an immunomodulatory imide drug (IMiD), more preferably selected from the group consisting of thalidomide, lenalidomide and pomalidomide. Further active ingredients can also be steroids.

In a preferred embodiment, the pharmaceutical composition is for use in the AL amyloidosis and said further active ingredient is selected from the group consisting of melphalan, proteasome inhibitors, steroids, thalidomide, lenalidomide, pomalidomide and cyclophosphamide.

The pharmaceutical composition according to the present invention may contain suitable pharmaceutical acceptable carriers, biologically compatible vehicles suitable for administration to an animal (for example, physiological saline) and optionally comprising auxiliaries (like excipients, stabilizers or diluents) which facilitate the processing of the active compounds into preparations which can be used pharmaceutical.

The pharmaceutical composition according to the present invention may be formulated in any acceptable way to meet the needs of the mode of administration. The use of biomaterials and other polymers for drug delivery, as well the different techniques and models to validate a specific mode of administration, are disclosed in literature.

Any accepted mode of administration can be used and determined by those skilled in the art. For example, administration may be by various parenteral routes such as subcutaneous, intravenous, intradermal, intramuscular, intraperitoneal, intranasal, transdermal. Enteral routes, such as for example oral, or rectal, are also provided.

"Therapeutically effective amount" is an amount effective to achieve the medically desirable result in the treated subject.

The compounds of the present invention may be prepared from readily available starting materials using the following general methods and procedures. It will be appreciated that where typical or preferred experimental conditions (i. e., reaction temperatures, time, moles of reagents, solvents, etc.) are given, other experimental conditions can also be used unless otherwise stated. Optimum reaction conditions may vary with the particular reactants or solvents used, but such conditions can be determined by one skilled in the art by routine optimisation procedures.

The composition of the invention will generally be administered in a suitable formulation. Such formulation takes the form of conventional pharmaceutical compositions.

Hence, pharmaceutical compositions comprising roneparstat and a pharmaceutically acceptable carrier, diluent or excipient for the use according to the present invention is also within the scope of the present invention. Such compositions can be prepared in a manner well known in the pharmaceutical art and comprise at least one active compound. A person skilled in the art is aware of a whole variety of such carrier, diluent or excipient compounds suitable to formulate a pharmaceutical composition.

Roneparstat together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous use). Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

Generally, each active ingredient is administered in a "pharmaceutically effective amount". The amount of the compound actually administered will typically be determined by a physician, in the light of the relevant circumstances, including the condition to be treated, the chosen route of administration, the actual compound or combination of compounds administered, other concomitant drug combinations, the age, body weight, and response of the individual patient, the severity of the patient's symptoms, and the like. Generally, an effective dose will be from 0.01 mg/kg to 100 mg/kg, preferably 0.05 mg/kg to 50 mg/kg. Compositions may be administered individually to a patient or may be administered in combination with other agents, drugs, hormones, irradiation or surgery. For any compound, the therapeutically effective dose can be estimated initially either in cell culture assays or in animal models, usually mice, rats, guinea pigs, rabbits, dogs, monkeys or pigs.

The animal model may also be used to determine the appropriate concentration range and route of administration. Such information can then be used to determine useful doses and routes for administration in humans. In calculating the Human Equivalent Dose (HED) it is recommended to use the conversion table provided by the FDA in Guidance for Industry and Reviewers document available from FDA. The pharmaceutical compositions of the invention can be administered by a variety of routes including oral, rectal, sublingual, transdermal, subcutaneous, intravenous, intramuscular, intrathecal, intraperitoneal, intranasal and locally on the diseased tissue after surgical operation.

The dose and mode of administration as well as the therapeutic posology will be determined by the physician according to his experience, severity of the disease, conditions of the patient and any other consideration pertaining to medical profession.

Roneparstat is administered to patients in need thereof at doses suggested by the physicians in the clinical practice. For example suitable subcutaneous doses are comprised between 100 and 600 mg per day, preferably between 200 and 400 mg per day (see for example Galli et al., 2018).

Depending on the intended route of delivery, Roneparstat is preferably formulated as parenteral, topical or oral compositions. The compositions for oral administration may take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include refilled, pre-measured ampoules or syringes of the liquid compositions or pills, tablets, capsules or the like in the case of solid compositions. In such compositions, the compound of the invention can be a minor or major component with the remainder being various vehicles or carriers and processing aids helpful for forming the desired dosing form. In the case of a lyophilized composition for injection Roneparstat is generally present as a major component of the composition (80-100% by weight). When reconstituted with water for injectable Roneparstat is generally at a concentration of from 50 to 200 mg/ml.

Dosage treatment may be a single dose schedule or a multiple dose schedule.

Liquid forms suitable for oral administration may include a suitable aqueous or nonaqueous vehicle with buffers, suspending and dispensing agents, colorants, flavors and the like.

Solid forms may include, for example, any of the following ingredients, or compounds of a similar nature: a binder such as microcrystalline cellulose, acacia, gum tragacanth, gelatine or polyvinyl-pyrrolidone; an excipient such as starch or lactose, a disintegrating agent such as alginic acid, Primogel, or potato or corn starch; a lubricant such as magnesium stearate, talc, polyethylene glycol or silica; a glidant such as colloidal silicon dioxide; a sweetening agent such as sucrose or saccharin; or a flavoring agent such as pepper-mint, methyl salicylate, or orange flavoring. The tablets may be coated according to methods well known from people skilled in the art of pharmaceutical practice.

Parenteral compositions are typically based upon injectable sterile saline or phosphate-buffered saline or other injectable carriers known in the art. Roneparstat can also be administered in sustained release forms or from sustained release drug delivery systems.

A description of representative sustained release materials as well as further materials and processing techniques are set out in Part 5 of Remington's Pharmaceutical Sciences, 20th Edition, 2000, Mack Publishing Company, Easton, Pennsylvania.

The present invention will be further illustrated by the following examples.

### EXAMPLES

### Transgenic model of AA amyloidosis

A well-known transgenic model is based on the use of the Tet-on system for transgene regulation, which is a binary system, that uses two transgenes. One transgene encodes an artificial transactivator (rtTA) protein; this transactivator binds to Tet-response elements (TREs) in the presence of a specific ligand, typically the orally bioavailable antibiotic doxycycline. In this case, the rtTA protein is expressed in hepatocytes. The second transgene (TRE-SAA) comprises the SAA gene under the control of a Tet-responsive minimal promoter, which is inactive under normal conditions. In mice carrying both transgenes, in the absence of doxycycline, the SAA concentrations are indistinguishable from those of non-transgenic control mice. In doxycycline-treated mice, the SAA concentrations achieved are similar to or exceed those of mice treated to induce a strong acute phase response. Doxycycline-induced SAA expression returns to baseline within 3 days, once the antibiotic has been eliminated. Notably, mice normally express two isoforms of SAA in approximately equal quantities, encoded by different closely related genes; only one of these isoforms contributes to AA amyloid. The transgenic mice only express the amyloidogenic isoform.

In the transgenic model, in which amyloid deposition was initiated and synchronised by amyloid enhancing factor (AEF) injection into SAA overexpressing mice, amyloid deposition has been observed 3 days and 7 days post-injection. In the 3 days group, amyloid loads were lower than in the 7 days group, but nevertheless all mice were amyloidotic.

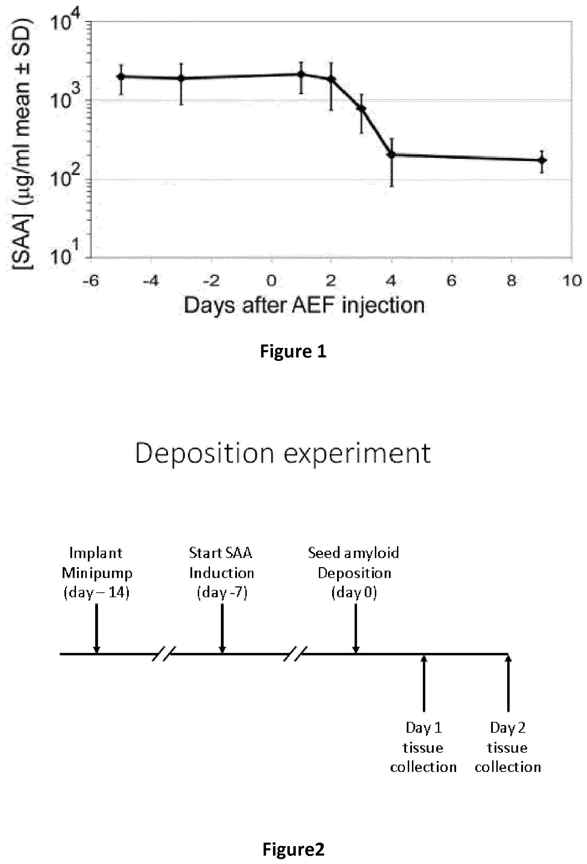

We found that when mice are actively depositing amyloid (after AEF injection), the circulating SAA concentration falls because of the high rate at which the protein is sequestered in amyloid. This effect was first significant 3 days after AEF injection and initiation of amyloid deposition (Figure 1).

In order to evaluate the effects of Roneparstat on amyloid we used both histological analysis and to assess the effects on circulating SAA concentrations.

### Example 1 - Amyloid deposits in the transgenic mouse model contain GAGs

### Materials and methods

Sections were cut from archival tissue blocks containing amyloidotic tissues from the transgenic mouse model; sections were stained with alcian blue to localise GAGs, and adjacent sections were stained with Congo red to identify amyloid. The tissues selected for this analysis included a range of different amyloid loads (10 mice), and 2 non-amyloidotic controls.

### Example 2 - Effect of Roneparstat on amyloid deposition

### Material and methods

The following protocol was used (see also Figure 2):
Day -14 implant minipump with Roneparstat or vehicle;
Day -7 Induce SAA by administration of doxycycline in drinking water at 2mg/ml (continuously until termination);
Day -1 Collect serum samples for SAA assay (induced, not depositing)
Day 0 Inject AEF i.v.
Day 2 Collect organs for histology and serum for SAA assay (cohort 1)
Day 3 Collect serum samples for SAA assay (induced, depositing; cohort 2)
Day 4 Collect organs for histology and serum for SAA assay (cohort 2)
   Cohort 1 and cohort 2 comprised 2 groups of 12 mice each, receiving either drug or vehicle.

### Analyses

Any effects on the rate of amyloid deposition was evident by differences in the amyloid loads between experimental and control groups, and by altered serum SAA concentration profile after AEF injection.

### Amyloid loads

Sections of spleen, liver, kidney and heart were stained with Congo red and amyloid loads were scored using polarizing microscopy. Scoring was performed blind by at least two experienced individuals, using the well-established, robust and reproducible six-point semi-quantitative scale (0-5) that covers the full dynamic range of amyloid loads.

Cohorts 1 and 2 were treated as independent experiments for the purpose of statistical analysis. The amyloid loads in each organ of the treatment and control groups was analyzed by two tailed Mann-Whitney tests.

### SAA concentrations

The SAA concentrations was analyzed by repeated measures two-way ANOVA.

### Results

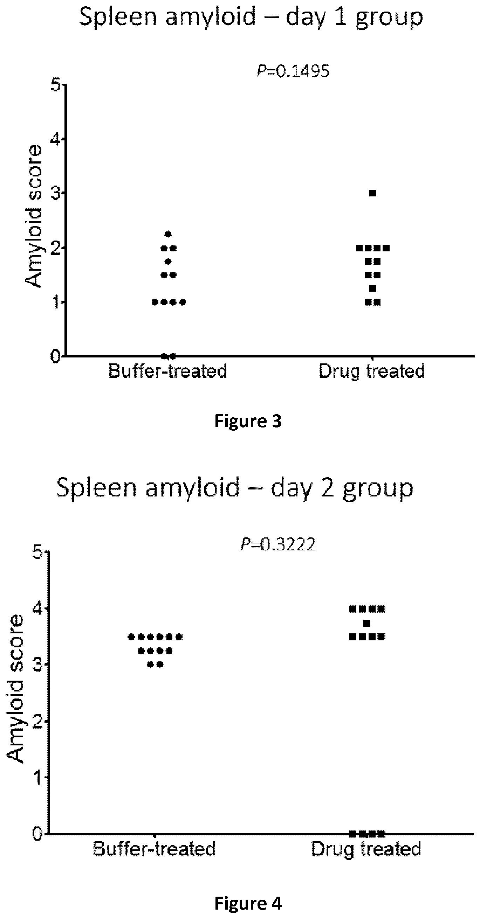

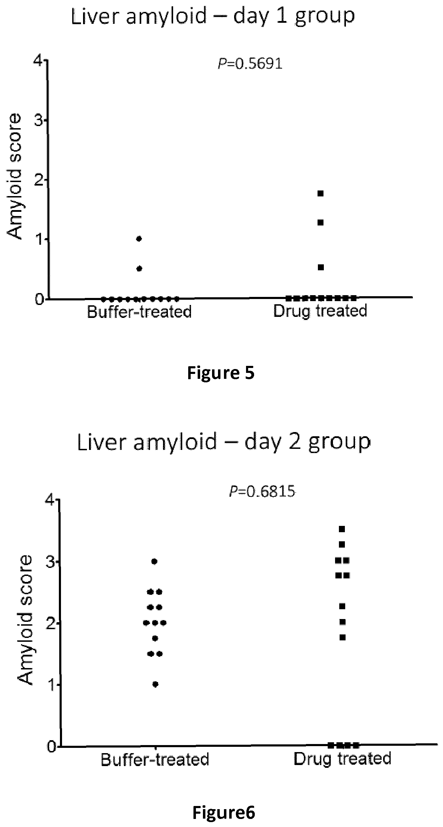

Results are shown in figures 3-6.

Unexpectedly, no negative effect on amyloid deposition was found in animals treated with Roneparstat with respect to controls.

### Example 2 - Effect of Roneparstat on amyloid regression

Although amyloid deposition can be extremely rapid, requiring only a few days, natural regression is a slow process, occurring over a period of months. Using the transgenic model, we previously found that two months after cessation of amyloid-inducing treatment, significant amyloid natural regression was evident, but that substantial amyloid deposits remained. Against this background, it was possible to detect either increased or decreased amyloid regression.

### Material and methods

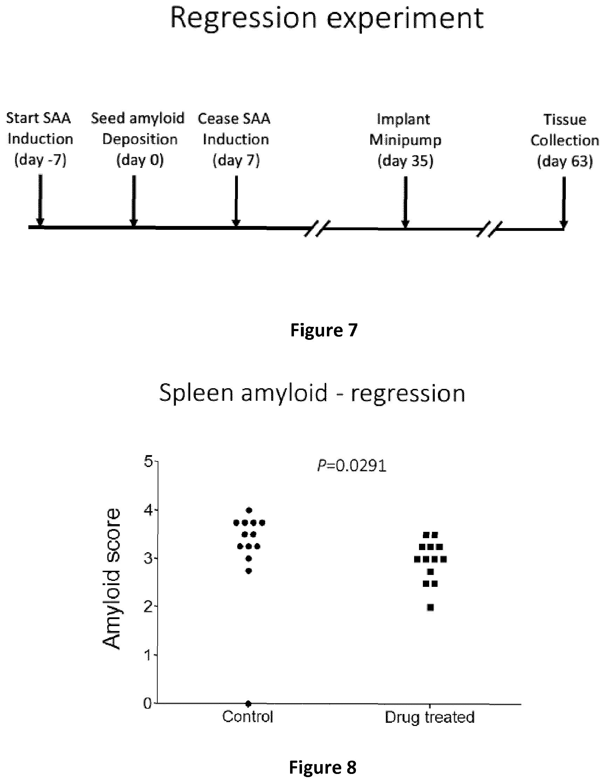

The following protocol was used (see also Figure 7):
Day -7 Induce SAA by administration of doxycycline in drinking water at 2mg/ml
Day -1 Collect serum samples for SAA assay (induced, not depositing)
Day 0 Inject AEF i.v.
Day 7 Cease doxycycline treatment
Day 35 Implant minipumps with Roneparstat or vehicle
Day 63 Collect organs for histology

Again, the group sizes were 12 mice. As above, the SAA assay conducted on the day -1 samples was used to determine whether any mice should be excluded from the analysis.

### Analyses

### Amyloid loads

Sections of spleen, liver, kidney and heart were stained with Congo red and amyloid loads were scored using polarizing microscopy. Scoring was performed blind by at least two experienced individuals, using the well-established, robust and reproducible six-point semi-quantitative scale (0-5) that covers the full dynamic range of amyloid loads.

The amyloid loads in each organ of the treatment and control groups was analyzed by two tailed Mann-Whitney tests.

### Results

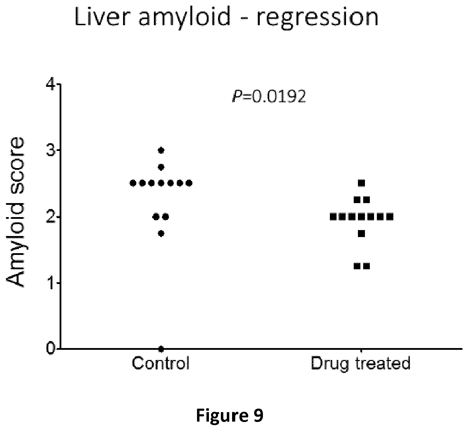

Results on amyloid regression are shown in Figures 8 and 9.

Unexpectedly, a positive effect on amyloid regression was observed in animals treated with Roneparstat with respect to controls. Indeed, amyloid score was lower in animals treated with Roneparstat than in controls.

### References

Desport E, Bridoux F, Sirac C, Delbes S, Bender S, Fernandez B, Quellard N, Lacombe C, Goujon JM, Lavergne D, Abraham J, Touchard G, Fermand JP, Jaccard A. Al amyloidosis. Orphanet J Rare Dis. 2012 Aug 21;7:54.
Galli M, Chatterjee M, Grasso M, Specchia G, Magen H, Einsele H, Celeghini I, Barbieri P, Paoletti D, Pace S, Sanderson RD, Rambaldi A, Nagler A. Phase I study of the heparanase inhibitor Roneparstat: an innovative approach for multiple myeloma therapy. Haematologica. 2018 Apr 26
Jendresen CB, Cui H, Zhang X, Vlodavsky I, Nilsson LN, Li JP. Overexpression of heparanase lowers the amyloid burden in amyloid-β precursor protein transgenic mice. J Biol Chem. 2015 Feb 20;290(8):5053-64.
Mahmood S, Palladini G, Sanchorawala V, Wechalekar A. Update on treatment of light chain amyloidosis. Haematologica. 2014 Feb;99(2):209-21.
Merlini G, Wechalekar AD, Palladini G. Systemic light chain amyloidosis: an update for treating physicians. Blood. 2013 Jun 27;121 (26):5124-30.
Real de Asúa D, Costa R, Galván JM, Filigheddu MT, Trujillo D, Cadiñanos J. Systemic AA amyloidosis: epidemiology, diagnosis, and management. Clin Epidemiol. 2014 Oct 29;6:369-77.
Wang B, Tan YX, Jia J, Digre A, Zhang X, Vlodavsky I, Li JP. Accelerated resolution of AA amyloid in heparanase knockout mice is associated with matrix metalloproteases. PLoS One. 2012;7(7):e39899.
Zhang X1, Li JP. Heparan sulfate proteoglycans in amyloidosis. Prog Mol Biol Transl Sci. 2010;93:309-34.

## Claims

1. Roneparstat or a pharmaceutically acceptable salt, or a hydrate or a solvate thereof for use for the treatment of amyloidosis.

2. Roneparstat for the use of claim 1, wherein amyloidosis is primary amyloid light-chain (AL) amyloidosis or secondary amyloid A (AA) amyloidosis.

3. A pharmaceutical composition comprising Roneparstat and at least one pharmaceutically acceptable vehicle and/or excipient for use for the treatment of amyloidosis.

4. The pharmaceutical composition for the use of claim 3, wherein said composition also comprises one or more further active ingredients.

5. The pharmaceutical composition for the use of claim 4, wherein said further active ingredient is a chemotherapeutic agent.

6. The pharmaceutical composition for the use of claim 5, wherein said chemotherapeutic agent is selected from the group consisting of cytotoxic agents, proteasome inhibitors, immunomodulatory drugs, topoisomerase inhibitors, kinase inhibitors, steroids and monoclonal antibodies, in particular directed towards highly expressed tumor associated antigens.

7. The pharmaceutical composition for the use of claim 6, wherein said cytotoxic agent, is selected from the group consisting of melphalan, irinotecan, rituximab and lapatinib or an alkylating agent, in particular cyclophosphamide.

8. The pharmaceutical composition for the use of claim 6 or 7, wherein said proteasome inhibitor is selected from the group consisting of bortezomib, carfilzomib and ixazomib.

9. The pharmaceutical composition for the use of claim 6, wherein said immunomodulatory drugs is an immunomoludatory imide, in particular selected from the group consisting of thalidomide, lenalidomide and pomalidomide.

10. The pharmaceutical composition for the use of anyone of claims 4-9, wherein said pharmaceutical composition is for use in the amyloid light-chain amyloidosis and said further active ingredient is selected from the group consisting of melphalan, proteasome inhibitors, steroids, thalidomide, lenalidomide, pomalidomide and cyclophosphamide.

11. Roneparstat for the use of claim 1 or 2, which is used in a combined therapy with melphalan and/or a proteasome inhibitor or in a proteasome inhibitor and/or melphalan containing regimens.
